(19) Europäisches Patentamt

European Patent Office

Office européen des brevets

(11) **EP 2 737 926 B1**

(12) **FASCICULE DE BREVET EUROPEEN**

(45) Date de publication et mention
de la délivrance du brevet:
**05.04.2017 Bulletin 2017/14**

(51) Int Cl.:
***A61N 5/06*** *(2006.01)* *A61B 17/00* *(2006.01)*

(21) Numéro de dépôt: **13193028.1**

(22) Date de dépôt: **15.11.2013**

(54) **Dispositif pour contrôler l'activation d'une substance photosensibilisante dans un tissu biologique**

Vorrichtung zum Steuern der Aktivierung eines Photosensibilisators in einem biologischen Gewebe

Apparatus for controlling the activation of a photosensitizer in a biological tissue

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2012 FR 1261339**

(43) Date de publication de la demande:
**04.06.2014 Bulletin 2014/23**

(73) Titulaire: **Université de Lorraine
54052 Nancy Cedex (FR)**

(72) Inventeurs:
• **Bastogne, Thierry
54180 Houdemont (FR)**
• **Tylcz, Jean-Baptiste
54000 Nancy (FR)**

(74) Mandataire: **Pontet Allano & Associes
Parc Les Algorithmes, Bâtiment Platon
CS 70003 Saint-Aubin
91192 Gif-sur-Yvette Cedex (FR)**

(56) Documents cités:
EP-A1- 1 637 182      EP-A1- 1 808 198
WO-A1-86/00515       WO-A1-99/06113
DE-A1- 19 721 489

EP 2 737 926 B1

## Description

### Domaine technique

La présente invention concerne un dispositif pour contrôler l'activation d'une substance photo-sensibilisante dans un tissu biologique.

Le domaine de l'invention est plus particulièrement mais de manière non limitative celui des thérapies photodynamiques, en particulier pour le traitement des cellules cancéreuses.

### Etat de la technique antérieure

Les thérapies photodynamiques (PDT) sont des techniques utilisées pour traiter des tissus biologiques et éliminer sélectivement des cellules. Elles sont utilisées notamment pour traiter des lésions cancéreuses et précancéreuses, en particulier de la peau. Elles sont également utilisées pour traiter d'autres affections telles que l'acné kystique, les kératoses actiniques, les tâches pigmentaires, la dégénération maculaire liées à l'âge, les rugosités, les vaisseaux dilatés, le psoriasis, ou pour des traitements anti-infectieux, des petites rides, ou l'épilation.

Le principe des thérapies photodynamiques est le suivant :

- dans un premier temps, un produit photosensibilisant (PS) est introduit dans les tissus biologiques ;
- la vitesse d'élimination du produit photosensibilisant étant variable en fonction de la nature des cellules et de leur environnement (vascularisation, ...), après un certain temps le produit restant dans le tissu biologique est essentiellement concentré autour des cellules cibles (par exemple dans la tumeur);
- le tissus biologique et le produit photosensibilisant sont alors illuminés par une lumière à une longueur d'onde appropriée ;
- le produit est conçu de telle sorte que sous l'effet de l'illumination il est « activé » et génère des espèces réactives de l'oxygène, tels que de l'oxygène singulet, à haute dose ;
- ces espèces réactives de l'oxygène ont pour effet de détruire ou de dégrader les molécules voisines. Ainsi, si le produit est concentré plus spécifiquement dans certaines zones du tissu biologique, ces zones sont sélectivement endommagées ou détruites.

Les produits photosensibilisants utilisés peuvent par exemple être des composés de la famille des porphyrines, ou des chlorines.

Les applications de la thérapie photodynamique concernent en particulier des affections et des cancers de la peau, ou des traitements de la dégénérescence maculaire due à l'âge. Dans ce cas les tissus sont facilement accessibles pour l'illumination.

La technique peut également être utilisée pour le traitement de tumeurs interstitielles, c'est-à-dire dans les tissus. Dans ce cas l'illumination peut être effectuée au moyen de fibres optiques introduites dans les tissus.

Un inconvénient majeur des thérapies photodynamiques, qui en limite le développement pour des applications cliniques, est leur faible reproductibilité *in-vivo.* En effet, le comportement et la réponse des tissus au traitement varient fortement d'un sujet à l'autre, ce qui entraine une grande incertitude et une grande variabilité sur les réponses thérapeutiques.

De manière habituelle, les traitements consistent à illuminer la zone à traiter pendant une durée prédéterminée, suivant une ou plusieurs séquences. Il n'y a donc pas de contrôle de l'efficacité en cours de traitement.

Les applications cliniques actuelles de la thérapie photodynamique reposent sur un modèle macroscopique statique simplifié du phénomène photophysique inhérent pour déterminer la concentration de produit photosensibilisant nécessaire et d'éclairement énergétique ou irradiance à appliquer :

$$[R] = k_s b \varepsilon \varphi_{\lambda_A} T [S_0] \Phi f, \qquad (Eq.\ 1)$$

où $[R]$ est une concentration d'espèces réactives de l'oxygène qui, si elles sont situées dans une région sensible des cellules cancéreuses, déclenchent une cascade de réactions conduisant à la mort de la cellule. $\varphi_{\lambda_A}$ désigne l'irradiance ou la puissance lumineuse par unité de surface reçue par la surface du tissu à traiter et $\lambda_A$ correspond à une longueur d'onde d'absorption du PS. $T$ est le temps d'exposition à la lumière et $[S_0]$ est la concentration intracellulaire d'agent photosensibilisant. $k_s$ est le coefficient de rétro-diffusion du tissu, $b$ est un rendement de conversion, $\varepsilon$ est le coefficient d'extinction de l'agent photosensibilisant et $\Phi$ est un rendement quantique de conversion du médicament en radicaux oxydants. $f$ désigne la fraction d'espèces réactives de l'oxygène générées attaquant les cibles sensibles des cellules, tandis que l'autre fraction (1-*f*) ne cause que des effets mineurs.

En dépit de son utilisation actuelle dans les applications cliniques, ce modèle est sujet à plusieurs controverses :

- l'Eq. 1 montre une simple réciprocité entre la concentration de l'agent photosensibilisant et la lumière, alors que plusieurs expériences ont montré des résultats contradictoires ;
- le rendement quantique Φ est en réalité une fonction du taux d'oxygénation de la tumeur. Ce dernier est souvent variable dans le temps et reste difficile à mesurer durant le traitement ;
- les sites intracellulaires endommagés par la PDT dépendent beaucoup de la localisation du PS dans les cellules. Les sites d'action de la PDT sont principalement les mitochondries, le reticulum endoplasmique, l'appareil de Golgi, les lysosomes et les lipides membranaires. Une re-localisation du PS peut se produire pendant la PDT. Certains sites sont plus critiques que d'autres mais ce facteur n'est pas pris en compte dans l'Eq. 1;
- plusieurs quantités impliquées dans cette équation sont en réalité variables dans le temps. En particulier, il a été montré qu'une réduction de la concentration du PS durant le traitement était un facteur non négligeable, mais absent de cette formule. Plus généralement, l'Eq. 1 n'est qu'une description statique du processus dynamique de la PDT.

Il est donc nécessaire de mettre au point des méthodes de dosimétrie pour améliorer la reproductibilité des réponses thérapeutiques de la PDT. Une telle méthode de dosimétrie doit permettre de déterminer un dosage des facteurs incidents (tels que la concentration locale en produit photosensibilisant, la densité des photons envoyés, le temps d'illumination, l'intervalle entre l'administration du produit et les phases d'illumination), pour obtenir un effet souhaité (par exemple la destruction sélective de certaines cellules).

La détermination d'une méthode de dosimétrie précise constitue une voie de recherche primordiale. Il existe plusieurs classes de dosimétrie potentiellement utilisables en PDT, et toutes reposent sur un modèle :

## La dosimétrie explicite

Ce type de technique implique la mesure des principaux composants de la thérapie, c'est-à-dire les doses de lumière, d'oxygène et la concentration intracellulaire de l'agent photosensibilisant. Ces trois constituants sont regroupés dans un modèle de dose des espèces réactives de l'oxygène produites, défini par l'Eq. 1. Cependant, aucun de ces éléments n'est mesurable facilement. En outre, d'autres inconvénients déjà évoqués précédemment, comme la variation des doses des trois éléments au cours du traitement, constituent autant de limites au développement de cette approche.

## La dosimétrie implicite

Ce type d'approche propose d'utiliser une seule grandeur dont l'évolution dépend de l'effet conjugué des trois variables de base de la PDT (doses de lumière, de produit administré et d'oxygène). Cette grandeur doit être en mesure de renseigner sur les dommages biologiques prévisibles et donc sur les issues thérapeutiques du traitement. Une variable intéressante pour jouer ce rôle est la concentration intratumorale de l'agent photosensibilisant (PS).

Une technique de mesure connue consiste à utiliser une méthode invasive, nécessitant l'exérèse de tumeurs xénogreffées chez des souris « nude » (avec un système immunitaire déficient) ayant reçu l'agent photosensibilisant, puis d'estimer les concentrations du PS avant et après illumination par chromatographie liquide haute performance. Cette approche fournit des résultats précis sur la concentration du PS et des photoproduits. Mais elle ne permet pas d'observer en temps réel l'évolution du PS durant le traitement.

## La mesure des réponses biologiques/biophysiques des tissus

Il s'agit d'une approche alternative pour suivre l'évolution du traitement et prédire les dommages sur les tissus comme les dommages vasculaires et les nécroses induites par le traitement.

Les méthodes utilisées dans ce but incluent la spectroscopie par impédance électrique, la mesure du flux sanguin par laser Doppler, l'analyse de la diffusion par spectroscopie de corrélation de fluorescence, la tomographie Doppler par cohérence optique, aussi bien que les méthodes d'imagerie moléculaire ou radiologique telles que la tomographie à émission de positron (PET). Il est encore difficile de savoir si ces techniques pourront prédire efficacement les réponses thérapeutiques de la PDT car elles nécessitent en particulier des changements rapides et significatifs des caractéristiques biologiques et spectrales des tissus.

On connaît par exemple le document EP 2 431 072 A1 qui divulgue un procédé de contrôle de l'illumination en thérapie photodynamique de tumeur interstitielle basé sur une mesure de propriétés optiques des tissus et une méthode de calcul pour déterminer l'état du tissu pendant le traitement.

<u>La dosimétrie directe</u>

Cette technique implique la mesure directe des concentrations des espèces réactives de l'oxygène et en particulier l'oxygène singulet. Comme pour l'approche de dosimétrie implicite, cette stratégie ramène le problème de dosimétrie à une seule dimension, celle de l'oxygène singulet mais directement cette fois. Plusieurs travaux ont montré des premiers résultats *in vivo* en laboratoire mais l'instrumentation actuelle requise pour ces travaux ne permet pas d'envisager des applications cliniques dans l'immédiat.

Le document EP 1 637 182 A1 décrit un dispositif de thérapie photodynamique comprenant un élément de commande d'une source lumineuse en fonction d'une information de fluorescence ou de phosphorescence déterminée au niveau d'une lésion en profondeur.

Le document DE 197 21 489 A1 décrit un dispositif de thérapie photodynamique comprenant une source lumineuse apte à s'adapter à la substance photosensibilisante utilisée.

Le document WO 99/06113 décrit un système de commande de la dosimétrie en thérapie photodynamique dans lequel la fluorescence est mesurée afin de déterminer la quantité de substance photosensibilisante produite, et en déduire l'instant auquel la thérapie doit stopper.

Le document EP 1 808 198 A1 décrit un dispositif de thérapie photodynamique comprenant un dispositif de commande prévu pour surveiller l'activation de la substance photosensibilisante dans un tissu sain situé devant une lésion à traiter.

Un objet de la présente invention est de proposer un dispositif d'ajustement automatique du rayonnement électromagnétique d'activation de l'agent photosensibilisant pendant une session de traitement en thérapie photodynamique *in-vivo*, et qui résolve les inconvénients de l'art antérieur.

Un autre objet de la présente invention est de proposer un dispositif d'ajustement automatique du rayonnement électromagnétique d'activation de l'agent photosensibilisant qui permette de contrôler de manière reproductible l'application d'un traitement en thérapie photodynamique.

Un autre objet de la présente invention est de proposer un dispositif d'ajustement automatique du rayonnement électromagnétique d'activation de l'agent photosensibilisant en thérapie photodynamique qui puisse être mise en oeuvre avec une instrumentation simple et adaptée aux applications *in-vivo.*

**Exposé de l'invention**

Un procédé exemplaire pour contrôler l'activation d'une substance photosensibilisante préalablement introduite dans un tissu biologique, comprenant une étape d'exposition de ladite substance photosensibilisante à un rayonnement électromagnétique d'activation de telle sorte à l'activer,
caractérisé en ce qu'il comprend en outre des étapes :

- d'obtention d'une information de luminescence issue de ladite substance photosensibilisante, et
- d'ajustement de l'exposition de la substance photosensibilisante au rayonnement électromagnétique d'activation de telle sorte que ladite information de luminescence suive une évolution temporelle correspondant à une consigne temporelle prédéterminée.

Il est entendu que le terme de luminescence regroupe tous modes d'émission de lumière dits « froids », tels que par exemple la chimiluminescence, la phosphorescence ou la fluorescence.

La substance photosensibilisante peut être introduite dans le tissu biologique notamment par injection, ou par simple contact dans le cas d'une surface accessible telle que la peau.

La consigne temporelle prédéterminée peut comprendre toutes sortes de profils de courbes, décrites par exemple par des équations (droites, courbes linéaires par morceaux, polynômes ...) ou des tableaux de points.

Cette consigne temporelle peut par exemple être issue de protocoles de mesure.

Le rayonnement électromagnétique d'activation peut comprendre un rayonnement ionisant (rayons X), et/ou un rayonnement non ionisant.

Suivant des modes de réalisation, le rayonnement électromagnétique d'activation peut comprendre un rayonnement lumineux d'activation avec des longueurs d'ondes optiques.

Suivant des modes de réalisation, le procédé peut comprendre en outre une étape de mesure d'une information de luminescence issue de l'oxygène singulet généré par l'activation de la substance photosensibilisante.

Cette information de luminescence peut être notamment issue d'une phosphorescence à une longueur d'onde de 1,27 $\mu$m de l'oxygène singulet qui est généré par des substances photosensibilisantes telles que les porphyrines ou les chlorines.

Suivant des modes de réalisation, le procédé peut comprendre en outre des étapes :

- d'exposition de la substance photosensibilisante à un rayonnement lumineux d'activation appliqué sous forme d'impulsions lumineuses d'activation, et
- de mesure de luminescence entre lesdites impulsions.

Cette exposition de la substance photosensibilisante à un rayonnement lumineux d'activation appliqué sous forme d'impulsions lumineuses séparées par des périodes d'obscurité permet ainsi d'effectuer des mesures de luminescence en temps réel, au cours du traitement, et y compris dans les cas où de telles mesures ne sont pas possibles en présence du rayonnement lumineux d'activation.

La mesure de luminescence peut comprendre des étapes :

- d'illumination de la substance photosensibilisante avec une lumière d'excitation à une longueur d'onde d'excitation,
- de mesure d'une intensité de fluorescence.

Dans ce cas, la mesure de luminescence comprend une mesure de fluorescence effectuée sur la substance photosensibilisante. L'évolution temporelle de cette fluorescence permet d'évaluer le phénomène de photoblanchiment de la substance photosensibilisante qui résulte de sa dégradation.

Le procédé peut comprendre en outre une étape de calcul d'un observateur de luminescence utilisant en entrée au moins une mesure de luminescence et une commande des impulsions lumineuses d'activation, et produisant en sortie une estimation de la luminescence.

De préférence, ces mesures de luminescence comprennent des mesures de fluorescence effectuées sur la substance photosensibilisante.

L'observateur de luminescence peut être modélisé sous la forme d'un observateur d'état comprenant une variable d'état correspondant à une estimation de la luminescence (ou de la fluorescence) associée à l'effet seul du traitement.

Cet observateur d'état peut être dérivé d'un modèle autorégressif avec entrées externes comme la structure de modèle ARX (« Auto Regressive model with eXternal inputs » en Anglais) ou un modèle de type représentation d'état.

L'observateur de luminescence peut comprendre des paramètres qui sont déterminés lors d'une étape préalable de calibration à partir de mesures expérimentales.

L'observateur de luminescence permet de réaliser une interpolation intelligente des mesures de luminescence ou de fluorescence. En effet, en pratique ces mesures ne peuvent pas être réalisées pendant les phases d'impulsions du rayon électromagnétique car ce dernier aveugle de par sa grande intensité le système de mesure de luminescence.

L'observateur de luminescence permet aussi de réduire très sensiblement l'intensité du bruit de mesure de luminescence.

L'observateur de luminescence permet ainsi d'améliorer considérablement le fonctionnement d'une boucle de régulation de la lumière d'activation.

En outre, l'observateur de luminescence est déterminé à partir de paramètres expérimentaux relativement faciles à obtenir. Une grande précision n'est pas nécessairement requise puisque l'observateur de luminescence peut être recalé périodiquement par les mesures de luminescence. Ainsi, ses paramètres peuvent être déterminés par exemple pour un type de tissu biologique donné, et applicables directement à différents sujets traités.

Le procédé peut comprendre en outre une étape de comparaison de l'estimation de la luminescence issue de l'observateur de luminescence avec la consigne temporelle de telle sorte à générer un signal d'erreur, lequel étant utilisé pour l'ajustement de l'exposition au rayonnement électromagnétique d'activation.

L'ajustement de l'exposition au rayonnement électromagnétique d'activation peut comprendre un ajustement de l'amplitude et/ou de la durée des impulsions lumineuses.

Ainsi, le procédé permet un asservissement en boucle fermée de l'exposition au rayonnement électromagnétique d'activation de telle sorte qu'une variable de mesure (la luminescence mesurée ou estimée par l'observateur d'état) suive une trajectoire prédéterminée (la consigne temporelle).

Le procédé peut être est mis en oeuvre avec une substance photosensibilisante dont l'activation produit des substances aptes à détruire des cellules. Il peut notamment être est mis en oeuvre avec des substances photosensibilisantes telles que les porphyrines ou les chlorines, qui libèrent de l'oxygène singulet.

Suivant un autre aspect, il est proposé un dispositif pour contrôler l'activation d'une substance photosensibilisante préalablement introduite dans un tissu biologique, comprenant :

- des moyens d'exposition aptes à permettre une exposition de ladite substance photosensibilisante à un rayonnement électromagnétique d'activation de telle sorte à l'activer,
- des moyens d'acquisition aptes à produire une information de luminescence issue de ladite substance photosensibilisante, et
- des moyens électroniques et de calcul aptes à contrôler les moyens d'exposition de telle sorte à permettre un ajustement de l'exposition de la substance photosensibilisante au rayonnement électromagnétique d'activation de telle sorte que ladite information de luminescence suive une évolution temporelle correspondant à une consigne temporelle prédéterminée.

Les moyens d'exposition peuvent comprendre une source lumineuse d'activation apte à produire des impulsions de lumière en fonction d'un signal de commande.

Les moyens d'acquisition peuvent comprendre un spectromètre ou un spectrofluorimètre.

Les moyens électroniques et de calcul peuvent implémenter une boucle de contrôle comprenant un observateur de luminescence,

- lequel observateur de luminescence utilisant en entrée au moins une mesure de luminescence et un signal de commande de la source lumineuse d'activation, et produisant en sortie une estimation de la luminescence,
- laquelle estimation de la luminescence étant utilisée pour générer un signal de commande de la source lumineuse d'activation.

**Description des figures et modes de réalisation**

D'autres avantages et particularités de l'invention apparaîtront à la lecture de la description détaillée des mises en oeuvre et des modes de réalisation nullement limitatifs, et des dessins annexés suivants :

- la Fig. 1 présente des résultats de simulation d'un processus de photoréaction, avec, Fig. 1(a) la concentration d'agent photosensibilisant PS à l'état fondamental (variable de sortie), Fig. 1(b) le signal d'irradiance (variable d'entrée), Fig. 1(c) la concentration d'oxygène singulet (variable d'état), et Fig. 1(d) la quantité cumulée d'oxygène singulet produite,
- la Fig. 2 présente un schéma synoptique de l'invention,
- la Fig. 3 présente un organigramme du procédé divulgué,
- La Fig. 4 illustre une comparaison entre des mesures de photoblanchiment *in-vitro* et une estimation du photoblanchiment obtenue avec le modèle d'état selon l'invention,
- la Fig. 5 illustre les performances de l'observateur de photoblanchiment, au travers de mesures simulées,
- la Fig. 6 présente des résultats de l'asservissement du photoblanchiment, en conditions *in-vitro*.

Modèle cytotoxique

Le processus de réaction à la base des thérapies photodynamiques est le suivant :

- un photosensibiliseur (PS) est introduit dans les tissus, par exemple par voie intraveineuse ou même par application directe, pour des applications sur la peau. Dans le cadre des modes de réalisation présentés, on considère que ce PS peut être par exemple une porphyrine ou une hématoprophyrine ;
- le PS se concentre plus ou moins sélectivement dans les tissus à détruire (par exemple les tissus tumoraux), ou il s'en élimine plus lentement ce qui produit le même résultat ;
- la zone à traiter est ensuite illuminée avec une lumière d'activation qui contient les longueurs d'onde nécessaires au déclanchement des réactions. Idéalement, ces longueurs d'onde correspondent à des bandes d'absorption du PS, ou du moins à un compromis entre les longueurs d'onde aptes à pénétrer les tissus biologiques (dans le rouge) et ces bandes d'absorption. Par exemple, pour des hématoporphyrines, l'illumination est souvent effectuée à des longueurs d'onde de l'ordre de 630-650 nm ;
- l'action de la lumière sur le PS entraîne la formation d'oxygène singulet $^1O_2$. Cette molécule est une forme d'oxygène de plus haute énergie que l'oxygène classique, qui est formée par excitation de ce dernier. C'est une molécule à courte durée de vie qui est très réactive vis-à-vis des composantes cellulaires et donc très toxique.

L'ensemble de ces processus entraîne la dégradation d'un grand nombre de constituants cellulaires, ce qui conduit à la mort des cellules par nécrose ou par apoptose.

Le processus photochimique à l'origine de la formation d'oxygène singulet est le suivant :

- le PS absorbe des photons de la lumière d'activation ;
- il passe alors d'un état singulet fondamental $S_0$ à un état singulet excité $S_1$ ;
- le retour à l'état initial $S_0$ peut s'opérer notamment en passant à un état triplet $T_1$ par conversion intersystème (ISC), donc une transition non radiative ;
- la durée de vie de cet état triplet $T_1$ étant relativement longue, la molécule de PS peut alors interagir avec des substrats situés dans son environnement proche, comme par exemple l'oxygène présent dans les tissus. Deux types de réactions, connues sous les noms de photoréactions de type I et II, sont possibles. Les photoréactions de type II sont prédominantes en PDT avec des PS de type porphyrines ;

- dans une photoréaction de type II, l'état triplet $T_1$ du PS réagit directement avec l'oxygène présent en lui transférant son excès d'énergie, ce qui fait passer l'oxygène de son état initial $^3O_2$ à son état singulet $^1O_2$.

Il est à noter que l'oxygène singulet formé peut également réagir avec le PS et le détruire. Cette dégradation, appelée photoblanchiment, est une conséquence des réactions de type I et II. Ce photoblanchiment conduit à une diminution de l'absorbance du PS, et donc à une diminution de son efficacité.

Le photoblanchiment met en jeu deux mécanismes :

- la photodégradation, qui correspond à une modification profonde de la structure du PS conduisant à la formation de photoproduits n'absorbant peu ou pas la lumière d'activation;
- la phototransformation qui correspond à une perte de l'absorbance et de la fluorescence du photosensibilisateur à certaines longueurs d'onde mais le chromophore est conservé.

Par ailleurs, si l'on irradie un PS à une longueur d'onde d'excitation adéquate, il est possible d'observer une fluorescence. Cette fluorescence correspond à un retour du PS à un état singulet excité $S_1$ vers l'état fondamental $S_0$ par émission de photons de fluorescence.

Cette fluorescence peut notamment être utilisée pour déterminer la localisation du photosensibilisateur dans les cellules, et pour vérifier sa complète élimination après traitement.

L'intensité de fluorescence est également affectée par le photoblanchiment. Ainsi, le phénomène de photoblanchiment peut être évalué par la mesure de la décroissance de l'intensité de fluorescence photoinduite du PS.

Dans le cadre de l'invention, un modèle non linéaire des processus de photoréaction à la base des thérapies photodynamiques (PDT) a été développé et décrit par une représentation d'état. Ce modèle a été obtenu à partir d'équations cinétiques, connues dans la littérature, qui décrivent les réactions de type II spécifiques à la PDT :

$$\begin{cases} \frac{d[S_0]}{dt} &= u_P + k_f[S_1] - k_{Pb}[S_0][\,^1O_2] + k_p[T_1] + k_T[T_1][\,^3O_2] - k_A u_L[S_0] \\ \frac{d[S_1]}{dt} &= k_A u_L[S_0] - (k_f + k_{ISC})[S_1] - k_{SM}[S_1][M] \\ \frac{d[T_1]}{dt} &= k_{ISC}[S_1] - k_p[T_1] - k_T[T_1][\,^3O_2] - k_{TM}[T_1][M] \\ \frac{d[\,^3O_2]}{dt} &= u_O - k_T[T_1][\,^3O_2] + k_l[\,^1O_2] \\ \frac{d[\,^1O_2]}{dt} &= k_T[T_1][\,^3O_2] - k_l[\,^1O_2] - k_{ox}[M][\,^1O_2] - k_{Pb}[S_0][\,^1O_2] \\ [M] &= [M]_0 \\ y &= \gamma[S_0] + v \\ v &\sim \mathcal{N}(0, s^2) \end{cases} \qquad \text{(Eq. 2)}$$

Les variables du modèle sont les suivantes:

$[S_0]$ : concentration de PS à l'état singulet fondamental (millimolaire, mM) ;
$[S_1]$ : concentration de PS à l'état singulet excité (mM) ;
$[T_1]$ : concentration de PS à l'état triplet excité (mM) ;
$[^3O_2]$ : concentration d'oxygène à l'état triplet initial (mM) ;
$[^1O_2]$ : concentration d'oxygène à l'état singulé excité (mM) ;
$[M]$ : concentration de cellules organiques (mM) ;
$u_P$ : vitesse d'absorption des molécules de PS dans les tissus (mM·s$^{-1}$) ;
$u_L$ : éclairement énergétique de l'illumination (W·cm$^{-2}$);
$u_O$ : vitesse d'absorption des molécules d'oxygène (mM·s$^{-1}$);

$y$ : intensité de fluorescence (unités arbitraires, u.a.). $x(t) = \left[[S_0], [S_1], [T_1], [\,^3O_2], [\,^1O_2], [M]\right]^T \in \mathbb{R}^6$ sont les variables d'état du modèle.

La pénétration des molécules photosensibilisantes (PS) dans les cellules est un processus avec une dynamique très lente, comparativement aux photoréactions. Aussi, la vitesse d'absorption des molécules photosensibilisantes $u_P$ peut être considérée comme nulle ($u_P$=0) pendant la durée du traitement, qui correspond à une période de temps relativement courte, inférieure à 1 heure.

La vitesse d'absorption de molécules d'oxygène $u_O(t)$ peut être supposée constante durant le traitement.

Le bruit de mesure $v(t_i)$ peut être considéré comme étant une séquence de réalisation de variables gaussiennes indépendantes et uniformément distribuées aux instants de mesure $\{t_i\}$, selon une loi normale $\mathcal{N}(0,\sigma^2)$.

Le modèle de l'Eq. 2 met également en oeuvre un ensemble de paramètres qui sont listés ci-après, avec les valeurs et les conditions initiales utilisées pour effectuer les calculs :

| Symbole | Définition | Valeur |
|---|---|---|
| $k_A$ | Constante d'absorption du photosensibiliseur pour un éclairement $u_L$ = 76mW·cm$^{-2}$ | 1 |
| $k_{Pb}$ | Constante de photoblanchiment | $1,6 \cdot 10^6$ mM$^{-1}$s$^{-1}$ |
| $k_T$ | Constante de vitesse bimoléculaire pour la réaction de $^3O_2$ avec $T_1$ | $10^5$ mM$^{-1}$s$^{-1}$ |
| $k_f$ | Constante de fluorescence pour la réaction $S_1 \rightarrow S_0$ | $2 \cdot 10^7$ S$^{-1}$ |
| $k_p$ | Constante de phosphorescence pour la réaction $T_1 \rightarrow S_0$ | 1250 $s^{-1}$ |
| $k_{ISC}$ | Constante de vitesse pour la réaction $S_1 \rightarrow T_1$ | $8 \cdot 10^7$ s$^{-1}$ |
| $k_l$ | Constante de luminescence pour la réaction $^1O_2 \rightarrow {}^3O_2$ | $10^5$ s$^{-1}$ |
| $k_{ox}$ | Constante bimoléculaire de la réaction d'oxydation entre $^1O_2$ et $M$ | $10^5$ mM$^{-1}$s$^{-1}$ |
| $\gamma$ | Coefficient de mesure | 43,15 u.a. |
| $[S_0]_0$ | Condition initiale pour $[S_0]$ | $8,5 \cdot 10^{-3}$ mM |
| $[S_1]_0$ | Condition initiale pour $[S_1]$ | 0 mM |
| $[T_1]_0$ | Condition initiale pour $[T_1]$ | 0 mM |
| $[^3O_2]_0$ | Condition initiale pour $[^3O_2]$ | $83 \cdot 10^{-3}$ mM |
| $[^1O_2]_0$ | Condition initiale pour $[^1O_2]$ | 0 mM |
| $[M]_0$ | Condition initiale pour $[M]$ | $830 \cdot 10^{-3}$ mM |

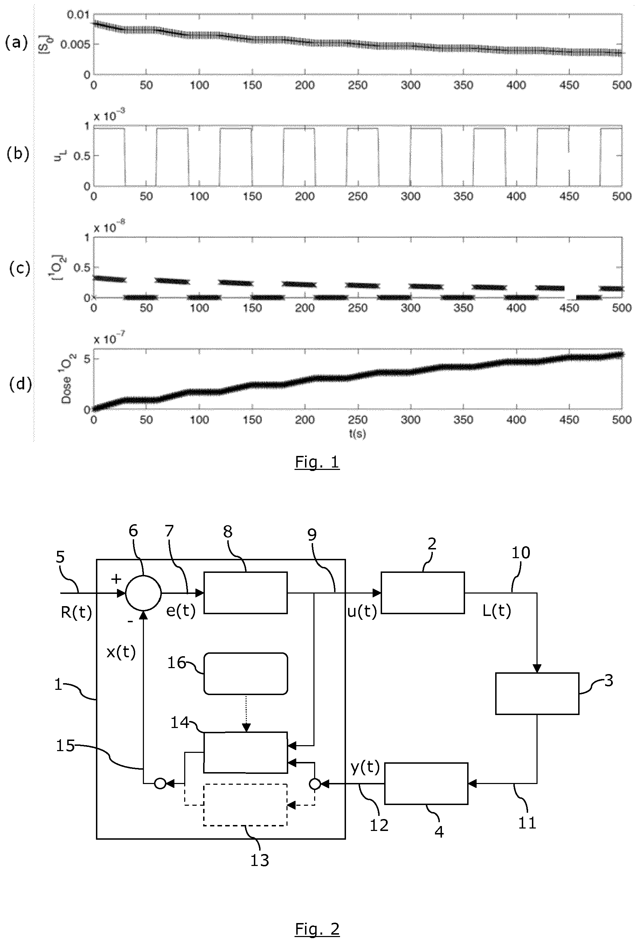

La Fig. 1 présente les résultats d'une simulation numérique d'un processus de photoréaction avec un agent photosensibilisant présent dans un tissu biologique. Cette simulation est calculée en implémentant le modèle de l'Eq. 2. Les axes des abscisses correspondent au temps t(s).

Le signal lumineux d'activation utilisé pour illuminer les tissus biologiques et le PS est présenté à la Fig. 1(b). Il s'agit d'un signal carré de période 60s, de rapport cyclique 1/2 et d'irradiance ou d'éclairement énergétique $u_L$=95 mW·cm$^{-2}$.

La Fig. 1(a) illustre l'évolution temporelle de la concentration $[S_0]$ de l'agent photosensibilisant à l'état singulet fondamental. Cette courbe correspond également à la courbe de photoblanchiment du PS.

La Fig. 1(c) illustre l'évolution de la concentration $[^1O_2]$ d'oxygène singulet produit par l'activation du PS par le signal lumineux d'activation.

La Fig. 1(d) illustre l'évolution de la quantité cumulée d'oxygène singulet produite par l'activation du PS par le signal lumineux d'activation.

Dans la mesure où l'oxygène singulet est l'espèce photocytotoxique véritablement à l'origine des dommages biologiques, une mesure de sa concentration au cours du traitement peut permettre de contrôler avec une grande précision l'application du traitement et ainsi de développer des techniques de dosimétrie capables d'amener une meilleure reproductibilité des résultats.

Une mesure directe de la concentration d'oxygène singulet peut être effectuée notamment en mesurant la luminescence produite à une longueur d'onde de 1,27 $\mu$m par cet oxygène singulet. Cependant, en pratique, cette concentration d'oxygène singulet est très difficilement mesurable dans des conditions *in-vivo*.

Les simulations de la Fig. 1 montrent également que la courbe de photoblanchiment du PS (Fig. 1(a)) est un bon indicateur de la production d'oxygène singulet (Fig. 1(d)). Ainsi suivant un mode de réalisation préférentiel de l'invention, c'est le signal de photoblanchiment qui est utilisé comme marqueur temporel de l'efficacité du traitement.

Comme expliqué précédemment, l'invention a pour de proposer une méthode de contrôle en boucle fermée qui permette, à partir de la surveillance en cours de traitement d'une variable témoin de l'efficacité du traitement (comme le photoblanchiment), d'ajuster les modalités d'application de l'irradiation pour que ce paramètre objectif suive une évolution temporelle prédéterminée. Ainsi, le traitement peut être adapté en temps réel de telle sorte que le signal de photoblanchiment suive une évolution prédéterminée en étant peu ou pas affecté par les variabilités biologiques entre les sujets, de telle sorte à améliorer la reproductibilité du traitement. La détermination de l'évolution temporelle ou de la courbe souhaitée dépend de considérations qui sortent du champ de la présente invention.

Comme expliqué précédemment, le photoblanchiment est estimé en mesurant une intensité de fluorescence du PS. Cette mesure ne peut pas être effectuée en même temps que l'illumination car le signal de fluorescence ne serait pas discernable.

Ainsi, dans le cadre de l'invention, l'illumination est appliquée sous la forme d'impulsions lumineuses telles que présentées à la Fig. 2(b), de telle sorte à pouvoir effectuer des mesures de photoblanchiment en temps réel entre ces impulsions.

Dosimétrie

Le fonctionnement du dispositif selon l'invention est illustré à la Fig. 2.

Le dispositif selon l'invention comprend une source de lumière d'activation 2 apte à générer une lumière d'activation 10.

Cette lumière d'activation 10 est dirigée par exemple au moyen de fibres optiques vers les tissus biologiques 3 de la zone à traiter, dans lesquels un agent photosensibilisant PS a été préalablement introduit. Comme expliqué précédemment, ce PS excité par la lumière d'activation provoque la génération d'espèces phototoxiques qui entraînent la destruction des cellules.

La source de lumière d'activation 2 est pilotée par un signal de commande d'activation 9 de forme temporelle $u(t)$, issu de la boucle d'asservissement 1. Ce signal de commande d'activation 9 se présente sous la forme d'une séquence d'impulsions telle qu'illustrée à la fig. 1(b). Ainsi, la lumière d'activation issue de la source 2 pilotée par le signal d'activation 9 est appliquée sous la forme d'une suite temporelle $L(t)$ d'impulsions lumineuses séparées par des moments d'extinction. La durée (et/ou l'amplitude) des impulsions lumineuses est contrôlée par le signal de commande 9.

Le dispositif selon l'invention comprend également un spectrofluorimètre 4 pour effectuer des mesures de photoblanchiment du PS. Ce spectrofluorimètre 4 comprend une source de lumière d'excitation qui émet une lumière à des longueurs d'onde aptes à exciter la fluorescence du PS. La source lumineuse peut par exemple être une diode laser à 410 nm. Cette lumière d'excitation est dirigée vers les tissus biologiques 3 de la zone à traiter au moyen d'au moins une fibre optique d'illumination. La fluorescence 11 générée par le PS est collectée par des fibres optiques regroupées en faisceau autour de la ou des fibre(s) optique(s) d'illumination, pour être amenée au spectromètre du spectrofluorimètre 4. Un spectre de fluorescence de forme $I(t,\lambda)$, où $\lambda$ est la longueur d'onde d'émission de fluorescence, est collecté pour plusieurs instants $t$ avec une période d'échantillonnage définie par l'utilisateur. L'intensité de fluorescence $y(t)$ émise par l'agent photosensibilisant peut alors être déterminée par la formule suivante :

$$y(t) = J(t, \lambda_2) - J(t, \lambda_1) \qquad\qquad (\text{Eq. 3})$$

où $j(t,\lambda)$ est une intégrale du spectre :

$$J(t, \lambda) = \int_{\lambda_0}^{\lambda} I(t, l)dl, \qquad\qquad (\text{Eq. 4})$$

et l'intervalle $[\lambda_1 ; \lambda_2]$ correspond à la largeur de bande d'émission de fluorescence de l'agent photosensibilisant. Cette intensité de fluorescence $y(t)$ est ensuite utilisée comme signal de mesure du photoblanchiment 12.

Le dispositif selon l'invention peut être mis en oeuvre pour traiter des affections à la surface de la peau ou d'une paroi biologique (oesophage, ...). Il peut également être mise en oeuvre pour traiter des affections ou des tumeurs interstitielles. Dans ce cas, la lumière d'activation 10 peut être amenée dans la zone à traiter 3 et la fluorescence 11 peut être collectée avec des fibres optiques insérées dans les tissus biologiques.

Comme expliqué précédemment, la mesure du photoblanchiment 12 ne peut être effectuée que lorsque la source d'activation 2 est éteinte, car dans le cas contraire la fluorescence 11 n'est pas discernable.

Le dispositif selon l'invention comprend enfin une boucle d'asservissement 1 implémentée dans un calculateur numérique. La fonction de cette boucle d'asservissement 1 est d'ajuster le signal de commande d'activation 9 de forme temporelle $u(t)$ de telle sorte que le signal de photoblanchiment 12 de forme temporelle $y(t)$ suive une consigne 5 de forme temporelle $R(t)$ prédéterminée.

Bien entendu, dans le cadre de l'invention cette consigne peut être de toute forme temporelle $R(t)$.

Dans la boucle d'asservissement 1, une grandeur d'état 15 de forme temporelle $x(t)$ qui dépend du signal de photoblanchiment 12 est comparée à la consigne 5 dans un comparateur 6, de telle sorte à générer un signal d'erreur de poursuite 7 de forme temporelle $e(t)$.

Le signal d'erreur de poursuite 7 est introduit dans un correcteur 8 qui génère un signal de commande 9 de la source d'activation 2 visant à minimiser cette erreur aux itérations suivantes.

Ce signal de commande d'activation 9 a une forme temporelle $u(t)$ qui correspond à une suite d'impulsions avec une période constante mais dont l'amplitude et/ou le rapport cyclique est ajusté en fonction du signal d'erreur de poursuite 7. Dans le cas d'un ajustement du rapport cyclique, qui est la solution mise en oeuvre dans le mode de réalisation présenté, ces impulsions sont obtenues à partir d'un signal sinusoïdal auquel est ajouté un offset dépendant de l'amplitude de l'erreur de poursuite $e(t)$. Ce signal sinusoïdal décalé en amplitude est ensuite transformé en impulsions de période constante et de rapport cyclique dépendant de l'offset calculé au moyen d'un comparateur, pour générer le signal de commande $u(t)$.

Suivant un premier mode de réalisation de l'invention, la boucle d'asservissement 1 comprend un interpolateur 13 apte à générer la grandeur d'état 15 à partir d'une interpolation, par une méthode connue de l'homme du métier, des mesures de photoblanchiment 12. Cette interpolation consiste à calculer des valeurs de la grandeur d'état $x(t)$ à partir d'un historique de mesures de photoblanchiment $y(t)$, éventuellement avec une fréquence d'échantillonnage plus élevée, et en effectuant un moyennage du bruit de mesure. Des interpolations de type polynômiales ou splines peuvent notamment être utilisées.

Suivant un second mode de réalisation préférentiel de l'invention, la boucle d'asservissement comprend un observateur d'état 14 dont le fonctionnement sera expliqué ci-après.

Bien entendu, l'interpolateur 13 et l'observateur d'état 14 peuvent être tous deux implémentés dans une boucle d'asservissement 1. Le choix de l'utilisation de l'un ou de l'autre peut alors être déterminé par un paramètre accessible à l'utilisateur.

En pratique, les mesures de fluorescence dans des conditions *in-vivo* sont des mesures difficiles. L'intensité de fluorescence 11 est très faible. Ces mesures nécessitent des temps d'intégration de l'ordre de la seconde, et demeurent très bruitées. En outre, les périodes d'illumination et d'obscurité du signal lumineux d'activation 10 sont de l'ordre de 1s à quelques dizaines de secondes. Sachant que les mesures de fluorescence 11 ne peuvent être effectuées que pendant les périodes d'obscurité du signal lumineux d'activation 10, il s'ensuit que le signal de photoblanchiment 12 qui peut être obtenu est en général très bruité et comprend peu d'échantillons.

Dans ces conditions, un interpolateur classique 13 risque de donner de mauvais résultats, et de générer une grandeur d'état 15 également très bruitée qui conduit à un mauvais fonctionnement global de la boucle de régulation 1.

L'estimation de la grandeur d'état 15 à partir des mesures de photoblanchiment 12 peut être considérablement améliorée en mettant en oeuvre un observateur d'état 14. Il s'agit d'un capteur logiciel qui est basé sur un modèle dynamique 16 du phénomène de photoblanchiment. Il accepte en entrée le signal de commande d'activation 9 et les mesures de photoblanchiment 12, et fournit en sortie une estimation de la grandeur d'état 15.

Le modèle dynamique 16 a pour fonction de simuler la chaine d'activation du PS et de mesure du photoblanchiment, qui comprend la génération du signal lumineux d'activation 10 par la source 2, les interactions avec les tissus biologiques 3 et la mesure du photoblanchiment 12 avec le spectrofluorimètre 4.

Le modèle biologique de l'Eq. 2 est difficilement applicable, car il dépend d'un grand nombre de paramètres difficiles à déterminer, et variables entre les sujets et les types de tissus biologiques.

Modèle dynamique simplifié

Il a donc été développé dans le cadre de l'invention un modèle dynamique simplifié, obtenu directement à partir de données expérimentales, et non pas à partir des équations théoriques des réactions photochimiques comme le modèle de l'Eq. 2.

Ce modèle dynamique simplifié permet à l'observateur d'état 14 de reconstruire en temps réel un signal de photoblanchiment "propre" à partir des données partielles et bruitées 12 mesurées pendant le traitement.

Dans le cas de la thérapie photodynamique, obtenir un modèle simplifié du comportement dynamique du phénomène de photoblanchiment est complexe. Il y a deux raisons à cela:

- le comportement non linéaire du phénomène de photoblanchiment visible sur les simulations de la Fig. 1(a) et 1(b). En effet, on observe clairement un comportement asymétrique de la réponse lorsque le signal d'excitation lumineuse est activé ou pas. Or, ce changement d'allure ne peut être décrit par un modèle linéaire classique;
- de plus, comme expliqué précédemment, on ne peut mesurer les spectres de fluorescence de l'agent photosensibilisant que pendant les phases où la lumière d'activation 10 est éteinte sinon elle sature le signal en raison de sa forte puissance. On est donc "aveugle" une grande partie du temps.

Ce modèle dynamique a été développé sur la base d'une structure ARX (« Auto Regressive Model with eXternal inputs » en Anglais ou modèle autorégressif avec des entrées externes). Cette structure ARX est définie par l'équation aux différences :

$$y(t) + a_1 y(t-1) + \cdots + a_{n_a} y(t - n_a)$$
$$= b_1 u(t - n_k) + \cdots + b_{n_b} u(t - n_k - n_b + 1) + v(t). \quad \text{(Eq. 5)}$$

$y(t)$ désigne la variable de sortie qui est ici le signal de photoblanchiment 12). $u(t)$ désigne la variable d'entrée qui est ici le signal de commande 9 de la source lumineuse d'activation 2. L'erreur $v(t)$ est une variable aléatoire dont la distribution suit une loi normale de moyenne nulle et de variance $\sigma^2$, $\mathcal{N}(0, \sigma^2)$. $t$ est la variable du temps qui représente les instants discrets échantillonnés avec une période d'échantillonnage $T_s$. $n_a$, $n_b$, $n_k$ sont des indices de structure, comprenant respectivement les ordres des polynômes des parties auto-régressives et externes ainsi que le retard de la sortie par rapport à l'entrée.

Pour décrire le comportement asymétrique du phénomène de photoblanchiment lorsque le signal d'excitation lumineuse est activé ou pas, on introduit un modèle ARX par morceaux, défini par les équations suivantes,

$$y(t) + a_{1,1} y(t-1) + \cdots + a_{1,n_a} y(t - n_a)$$
$$= b_{1,1} u(t - n_k) + \cdots + b_{1,n_b} u(t - n_k - n_b + 1) + v_1(t) \quad \text{(Eq. 6)}$$

si $\delta u(t) \geq 0$, et

$$y(t) + a_{2,1} y(t-1) + \cdots + a_{2,n_a} y(t - n_a)$$
$$= b_{2,1} u(t - n_k) + \cdots + b_{2,n_b} u(t - n_k - n_b + 1) + v_2(t) \quad \text{(Eq. 7)}$$

si $\delta u(t) \leq 0$.

$$\delta u(t) = u(t) - u(t-1)$$

correspond aux variations du signal de commande 9. $v_1(t)$ et $v_2(t)$ sont deux processus Gaussiens centrés indépendants et identiquement distribués.

Dans le cas présent, on peut se limiter à $n_a = n_b = n_k = 1$.

Les Eq. 6 et Eq. 7 peuvent être regroupées au sein d'une même relation,

$$y(t) + a_{1,1} y(t-1) + \left(a_{2,1} - a_{1,1}\right) y(t-1) b(t)$$
$$= b_{1,1} u(t-1) + \left(b_{2,1} - b_{1,1}\right) u(t-1) b(t) + v_1(t) + \left(v_2(t) - v_1(t)\right) b(t). \quad \text{(Eq. 8)}$$

La variable de séquencement $b(t)$ est une variable binaire nécessaire à la sélection des deux modèles linéaires. Elle est définie de la manière suivante :

$$\begin{cases} b(t) = 0 & si \quad \delta u(t) > 0 \\ b(t) = 1 & si \quad \delta u(t) < 0 \\ b(t) = b(t-1) & si \quad u(t) = 0 \end{cases} \quad \text{(Eq. 9)}$$

Le modèle de l'Eq. 8 peut être transformé en une représentation d'état à temps discret avec la variable d'état $x(t)$ :

$$x(t + 1) = -a_{1,1}x(t) + \boldsymbol{B}\,\boldsymbol{u}(t) \qquad\qquad \text{(Eq. 10)}$$

$$y(t) = x(t) + v(t), \qquad\qquad \text{(Eq. 11)}$$

où $\boldsymbol{B}$ = (-$a_{2,1}$ + $a_{1,1}$ $b_{1,1}$ $b_{2,1}$ - $b_{1,1}$), $\boldsymbol{u}^{T}(t)$= ($x(t)b(t)$ $u(t)$ $u(t)b(t)$), et $v(t) \sim \mathcal{N}(0, \sigma^2)$.

Dans le contexte de l'invention, on peut imposer que le comportement attendu soit constant durant les phases où la lumière d'activation est éteinte. Ce comportement peut être décrit par un modèle d'intégrateur avec un gain nul. En d'autres termes, cela revient à fixer $a_{2,1}$ = -1 et $b_{2,1}$ = 0.

Finalement, pour décrire complètement le modèle, il reste les paramètres $a_{1,1}$ et $b_{1,1}$ à déterminer. Ces paramètres peuvent être estimés à partir de mesures expérimentales réalisées dans des conditions proches des conditions d'application du traitement, par exemple lors d'expérimentations *in-vivo* sur des animaux.

La détermination de ces paramètres peut être effectuée avec le montage de la Fig. 2, dans une phase d'identification effectuée en boucle ouverte, en exploitant des mesures de photoblanchiment $y(t)$ obtenues en réponse à un signal de commande $u(t)$ connu. Les paramètres $a_{1,1}$ et $b_{1,1}$ sont ajustés pour que les Eq. 10 et Eq. 11 décrivent au mieux les mesures.

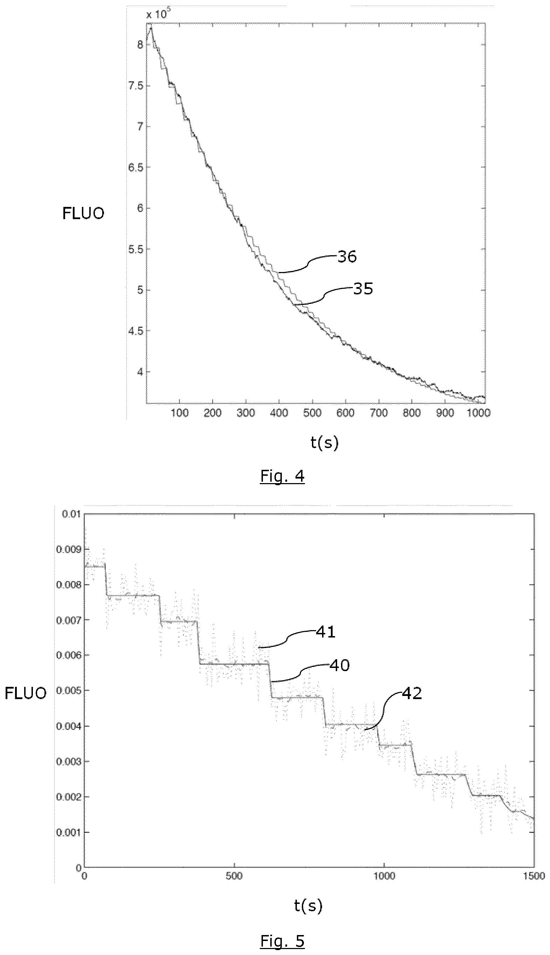

La Fig. 4 illustre la qualité d'ajustement de ce type de modèle sur des données de photoblanchiment obtenues en condition *in-vitro.* La courbe 35 représente des mesures de photoblanchiment $y(t)$ obtenues en réponse à un signal de commande $u(t)$, et la courbe 36 représente la réponse du modèle des Eq. 10 et Eq. 11 dans ces conditions. La correspondance entre la réponse du modèle et les mesures (coefficient de détermination) est de l'ordre de 95%.

Les Eq. 10 et Eq. 11 constituent ainsi le modèle dynamique 16 du phénomène de photoblanchiment, donc on peut voir qu'il peut être obtenu de manière simple en faisant un minimum d'hypothèses sur des paramètres physiologiques.

On peut également en déduire directement un modèle pour l'observateur d'état 14 comme suit :

$$x(t + 1) = -a_{1,1}x(t) + \boldsymbol{B}\,\boldsymbol{u}(t) + K\varepsilon(t) \qquad\qquad \text{(Eq. 12)}$$

$$\hat{y}(t) = x(t) \qquad\qquad \text{(Eq. 13)}$$

$$\varepsilon(t) = y(t) - \hat{y}(t), \qquad\qquad \text{(Eq. 14)}$$

Avec $\hat{y}(0)$ = $y(0)$. $\hat{y}(t)$ est le photoblanchiment estimé par l'observateur d'état 14, et $K$ un facteur multiplicatif (gain d'innovation).

Il est à noter que ce modèle est également en mesure de prendre en compte le photoblanchiment du PS dû à la lumière d'excitation émise par le spectrocolorimètre 4 pour générer la fluorescence.

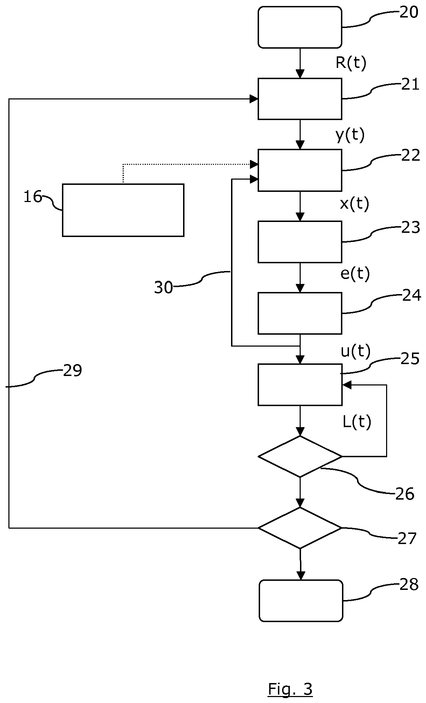

Le déroulement des étapes du procédé d'application de la PDT est illustré à la figure 3 :

- étape 20 : dans un premier temps, une trajectoire de photoblanchiment $R(t)$ prédéterminée est programmée ;
- étape 21 : une mesure de photoblanchiment est effectuée au moyen du spectrofluorimètre 4 pour obtenir une valeur de photoblanchiment mesurée $y(t)$ ;
- étape 22 : la variable d'état $x(t)$ (qui correspond à une valeur estimée de photoblanchiment $\hat{y}(t)$) est calculée par l'observateur d'état 14, en prenant en compte la dernière valeur du signal de commande de la source lumineuse d'activation $u(t)$ ;
- étape 23 : l'erreur de poursuite $e(t)$ est calculée ;
- étape 24 : le signal de commande de la source lumineuse d'activation u(t) est calculé par le correcteur 8 ;
- étape 25 : le signal lumineux d'activation $L(t)$ est appliqué par la source d'activation 2 à la zone à traiter 3 sous la forme d'une impulsion l'illumination dont la durée est déterminée par le signal de commande $u(t)$ ;
- étape 26 : le système attend la fin de l'impulsion lumineuse $L(t)$ ;
- étape 27 : si la trajectoire de photoblanchiment $R(t)$ est complètement parcourue, le traitement est arrêté (étape 28). Dans le cas contraire l'exécution de l'algorithme est poursuivie de manière itérative à l'étape 21 par une nouvelle mesure de photoblanchiment (retour à l'étape 21).

Il est à noter que cet algorithme comprend deux boucles imbriquées :

- une première boucle de mesure 29 entre les étapes 27 et 21 qui est cadencée par les mesures de photoblanchiment ;
- une deuxième boucle de mesure 30 entre les étapes 24 et 22 qui permet de prendre en compte les variations du signal de commande $u(t)$ dans les estimations du photoblanchiment $\hat{y}(t)$) fournies par l'observateur d'état 14.

La boucle de contre réaction 30 peut ainsi être exécutée à une cadence très supérieure à la boucle de mesure 29 (par exemple de l'ordre de 10 fois) en se basant sur des estimations produites par l'observateur à cadence régulière, même lorsque les mesures de photoblanchiment sont impossibles (durant l'illumination). Ainsi, le bruit dans la boucle de contre réaction peut être fortement diminué.

La période de la boucle de mesure 29 détermine la période du signal de commande $u(t)$. La durée des impulsions de ce signal de commande $u(t)$ ou son rapport cyclique est ajustée par la boucle de contre réaction 30.

Enfin, l'observateur d'état 14 peut être « recalé » à chaque itération de la boucle de mesure, ce qui permet de relâcher considérablement les contraintes sur la précision nécessaire pour le modèle dynamique 16.

La Fig. 5 illustre les performances de l'observateur de photoblanchiment. La courbe 40 est une courbe de photoblanchiment théorique calculée avec le modèle cytotoxique de l'Eq. 2. Cette courbe simule, de manière idéale et sans bruit de mesure, un processus d'asservissement du photoblanchiment effectué selon l'invention, qui suit une trajectoire de photoblanchiment $R(t)$ qui est une droite. A partir de cette courbe, des mesures bruitées 41 ont été générées en y rajoutant du bruit blanc, pour simuler des mesures de photoblanchiment $y(t)$ telles qu'elles auraient été obtenues avec le spectrofluorimètre 4 dans des conditions réelles. La courbe 42 présente les valeurs estimées de photoblanchiment $\hat{y}(t)$ produites par l'observateur d'état 14 à partir de la commande $u(t)$ et des mesures bruitées $y(t)$ dans ces conditions. On observe bien la capacité de l'observateur d'état 14 à estimer précisément la courbe du photoblanchiment théorique 40 malgré le bruit de mesure.

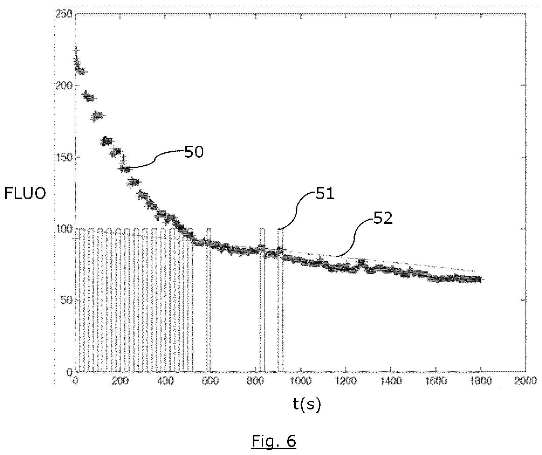

La Fig. 6 présente des mesures de l'asservissement du photoblanchiment obtenu avec le dispositif selon l'invention, dans des conditions *in-vitro.* La courbe 52 correspond à la trajectoire de photoblanchiment souhaitée $R(t)$, la courbe 50 correspond aux mesures de photoblanchiment $y(t)$, et la courbe 51 correspond au signal de commande de l'activation $u(t)$. On peut observer que malgré un écart important au départ entre la consigne $R(t)$ et les mesures $y(t)$, le système est capable de faire converger rapidement puis d'asservir le photoblanchiment au plus près de la trajectoire de consigne.

Suivant des variantes de modes de réalisation, la source de lumière d'activation 2 et le correcteur 8 peuvent être conçus de telle sorte à générer :

- des impulsions de lumière 10 d'amplitude variable en fonction de l'erreur de poursuite 7. Ces impulsions peuvent être de rapport cyclique constant (par exemple de 50%) et de période constante ;
- des impulsions de lumière 10 de durée et de période variable en fonction de l'erreur de poursuite 7.

Bien sûr, l'invention n'est pas limitée aux exemples qui viennent d'être décrits et de nombreux aménagements peuvent être apportés à ces exemples sans sortir du cadre de l'invention.

## Revendications

1. Dispositif pour contrôler l'activation d'une substance photosensibilisante préalablement introduite dans un tissu biologique (3), la substance photosensibilisante étant choisie parmi les porphyrines et les chlorines, le dispositif comprenant :

   - des moyens d'exposition (2) aptes à permettre une exposition de ladite substance photosensibilisante à un rayonnement électromagnétique d'activation (10) de telle sorte à l'activer, lesquels moyens d'exposition comprennent une source lumineuse d'activation (2) apte à produire des impulsions de lumière (10) en fonction d'un signal de commande (9), et
   - des moyens d'acquisition (4) aptes à produire une information de luminescence (11, 12) issue de ladite substance photosensibilisante,

   le dispositif étant **caractérisé en ce qu'**il comprend, en outre :

   - des moyens électroniques et de calcul aptes à contrôler les moyens d'exposition (2) de telle sorte à permettre un ajustement de l'exposition de la substance photosensibilisante au rayonnement électromagnétique d'activation (10) de telle sorte que ladite information de luminescence (11, 12) suive une évolution temporelle correspondant à une consigne temporelle prédéterminée (5),

les moyens électroniques et de calcul implémentant une boucle d'asservissement (1) comprenant :

- un observateur de luminescence (14) utilisant en entrée au moins une mesure de luminescence (12) et un signal de commande (9) de la source lumineuse d'activation (2), et produisant en sortie une estimation de la luminescence (15),
- des moyens (6) pour effectuer une comparaison de l'estimation de la luminescence (15) issue de l'observateur de luminescence (14) avec la consigne temporelle (5) de telle sorte à générer un signal d'erreur (7),
- un correcteur (8) apte à générer ledit signal de commande (9) de la source lumineuse d'activation (2) en utilisant le signal d'erreur (7).

2. Le dispositif de la revendication 1, qui comprend des moyens d'acquisition (4) aptes à produire une information de luminescence (11, 12) issue de l'oxygène singulet généré par l'activation de la substance photosensibilisante.

3. Le dispositif de la revendication 1, qui comprend des moyens d'acquisition (4) aptes à mesurer une intensité de fluorescence de la substance photosensibilisante entre les impulsions de lumière (10) de la source lumineuse d'activation (2).

4. Le dispositif de la revendication 3, qui comprend des moyens d'acquisition (4) comprenant :

- Une source de lumière d'excitation pour émettre une lumière à des longueurs d'onde d'excitation aptes à exciter la fluorescence de la substance photosensibilisante,
- des moyens de mesure d'une intensité de fluorescence (11).

5. Le dispositif de la revendication 4, dans lequel les moyens d'acquisition (4) comprennent un spectromètre ou un spectrofluorimètre.

6. Le dispositif de l'une des revendications précédentes, qui comprend un observateur de luminescence modélisé sous la forme d'un observateur d'état (14) comprenant une variable d'état (15) correspondant à une estimation de la luminescence.

7. Le dispositif de la revendication 6, dans lequel l'observateur d'état (14) est dérivé d'un modèle autorégressif (16) avec entrées externes ARX, ou d'un modèle de type représentation d'état.

8. Le dispositif de l'une des revendications précédentes, dans lequel l'observateur de luminescence (14) comprend des paramètres qui sont déterminés par calibration à partir de mesures expérimentales.

9. Le dispositif de l'une des revendications précédentes, dans lequel le correcteur (8) est apte à générer un signal de commande (9) qui correspond à une suite d'impulsions avec une période constante et dont l'amplitude et/ou le rapport cyclique est ajusté en fonction du signal d'erreur (7).

10. Le dispositif de l'une des revendications précédentes, qui est mis en oeuvre avec une substance photosensibilisante dont l'activation produit des substances aptes à détruire des cellules.

**Patentansprüche**

1. Vorrichtung zum Steuern der Aktivierung eines zuvor in ein biologisches Gewebe (3) eingeführten Photosensibilisators, wobei der Photosensibilisator ausgewählt ist aus Porphyrinen und Chlorinen, wobei die Vorrichtung enthält:

- Bestrahlungseinrichtungen (2), die dazu geeignet sind, eine Bestrahlung des Photosensibilisators mit einer elektromagnetischen Aktivierungsstrahlung (10) zu gestatten, so dass er aktiviert wird, wobei die Bestrahlungseinrichtungen eine Aktivierungslichtquelle (2) enthalten, die dazu geeignet ist, Lichtimpulse (10) in Abhängigkeit von einem Steuersignal (9) zu erzeugen, und
- Erfassungseinrichtungen (4), die dazu geeignet sind, eine von dem Photosensibilisator stammende Lumineszenzinformation (11, 12) zu erzeugen,

wobei die Vorrichtung **dadurch gekennzeichnet ist, dass** sie ferner enthält:

- elektronische Einrichtungen und Recheneinrichtungen, die dazu geeignet sind, die Bestrahlungseinrichtungen (2) so zu steuern, dass sie eine Anpassung der Bestrahlung des Photosensibilisators mit der elektromagnetischen Aktivierungsstrahlung (10) gestatten, so dass die Lumineszenzinformation (11, 12) einem zeitlichen Verlauf folgt, der einer vorbestimmten zeitlichen Vorgabe (5) entspricht,

wobei die elektronischen und die Recheneinrichtungen, die eine Regelschleife (1) implementieren, enthalten:

- ein Lumineszenzbeobachtungsglied (14), das am Eingang zumindest eine Lumineszenzmessung (12) und ein Steuersignal (9) der Aktivierungslichtquelle (2) verwendet und am Ausgang eine Abschätzung der Lumineszenz (15) erzeugt,
- Einrichtungen (6) zum Durchführen eines Vergleichs der von dem Lumineszenzbeobachtungsglied (14) stammenden Lumineszenzabschätzung (15) mit der zeitlichen Vorgabe (5), so dass ein Fehlersignal (7) erzeugt wird,
- einen Korrekturglied (8), das dazu geeignet ist, das Steuersignal (9) der Aktivierungslichtquelle (2) unter Verwendung des Fehlersignals (7) zu erzeugen.

2. Vorrichtung nach Anspruch 1, enthaltend Erfassungseinrichtungen (4), die dazu geeignet sind, eine Lumineszenzinformation (11, 12) zu erzeugen, die von dem Singulett-Sauerstoff stammt, der durch die Aktivierung des Photosensibilisators erzeugt wird.

3. Vorrichtung nach Anspruch 1, enthaltend Erfassungseinrichtungen (4), die dazu geeignet sind, eine Fluoreszenzintensität des Photosensibilisators zwischen den Lichtimpulsen (10) der Aktivierungslichtquelle (2) zu messen.

4. Vorrichtung nach Anspruch 3, enthaltend Erfassungseinrichtungen (4), die enthalten:

- eine Anregungslichtquelle zum Emittieren von Licht mit Anregungswellenlängen, die dazu geeignet sind, die Fluoreszenz des Photosensibilisators anzuregen,
- Einrichtungen zum Messen einer Fluoreszenzintensität (11).

5. Vorrichtung nach Anspruch 4, wobei die Erfassungseinrichtungen (4) ein Spektrometer oder ein Spektrofluorometer enthalten.

6. Vorrichtung nach einem der vorangehenden Ansprüche, enthaltend ein Lumineszenzbeobachtungsglied, das in Form eines Zustandsbeobachtungsglieds (14) mit einer Zustandsvariablen (15) ausgeführt ist, die einer Abschätzung der Lumineszenz entspricht.

7. Vorrichtung nach Anspruch 6, wobei das Zustandsbeobachtungsglied (14) von einem autoregressiven Modell (16) mit externen ARX-Eingängen oder von einem Modell vom Typ Zustandsdarstellung abgeleitet ist.

8. Vorrichtung nach einem der vorangehenden Ansprüche, wobei das Lumineszenzbeobachtungsglied (14) Parameter enthält, die durch Kalibrierung ausgehend von experimentellen Untersuchungen bestimmt sind.

9. Vorrichtung nach einem der vorangehenden Ansprüche, wobei das Korrekturglied (8) dazu geeignet ist, ein Steuersignal (9) zu erzeugen, das einer Folge von Impulsen mit einer konstanten Periode entspricht, wobei die Amplitude und/oder das Tastverhältnis in Abhängigkeit von dem Fehlersignal (7) angepasst wird.

10. Vorrichtung nach einem der vorangehenden Ansprüche, die mit einem Photosensibilisator betrieben wird, dessen Aktivierung Stoffe erzeugt, die dazu geeignet sind, Zellen zu zerstören.

## Claims

1. Device for controlling the activation of a photosensitizer previously introduced into a biological tissue (3), the photosensitizer being selected from the porphyrins and the chlorines, the device comprising:

- exposure means (2) capable of allowing an exposure of said photosensitizer to an activating electromagnetic radiation (10) in order to activate it, said exposure means comprising an activation light source (2) capable of producing light pulses (10) as a function of a control signal (9), and
- acquisition means (4) capable of producing luminescence information (11, 12) originating from said photosensitizer,

the device being **characterized in that** it comprises, moreover:

- electronic and calculation means capable of controlling the exposure means (2) so as to allow an adjustment of the exposure of the photosensitizer to the activating electromagnetic radiation (10) so that said luminescence information (11, 12) develops overtime corresponding to a predetermined time setting (5),

the electronic and calculation means implementing a feedback loop (1) comprising:

- a luminescence observer (14) using as input at least one luminescence measurement (12) and a control signal (9) of the light activation source (2), and producing as output an estimation of the luminescence (15),
- means (6) for carrying out a comparison of the estimation of luminescence (15) originating from the luminescence observer (14) with the time setting (5) so as to generate an error signal (7),
- a corrector (8) capable of generating said control signal (9) of the light activation source (2) using the error signal (7).

2. The device according to claim 1, which comprises acquisition means (4) capable of producing luminescence information (11, 12) originating from the oxygen singlet generated by the activation of the photosensitizer.

3. The device according to claim 1, comprising acquisition means (4) capable of measuring a fluorescence intensity of the photosensitizer between the light pulses (10) of the activation light source (2).

4. The device according to claim 3, which comprises acquisition means (4) comprising:

- an excitation light source for emitting light at excitation wavelengths capable of exciting the fluorescence of the photosensitizer,
- means of measuring a fluorescence intensity (11).

5. The device according to claim 4, in which the acquisition means (4) comprise a spectrometer or a spectrofluorimeter.

6. The device according to one of the preceding claims, which comprises a luminescence observer modelled in the form of a state observer (14) comprising a state variable (15) corresponding to an estimation of the luminescence.

7. The device according to claim 6, in which the state observer (14) is derived from an auto regressive model (16) with external ARX inputs, or a model of the state representation type.

8. The device according to one of the preceding claims, in which the luminescence observer (14) comprises parameters that are determined by calibration from experimental measurements.

9. The device according to one of the preceding claims, in which the corrector (8) is capable of generating a control signal (9) which corresponds to a sequence of pulses with a constant period and of which the amplitude and/or the duty cycle is adjusted as a function of the error signal (7).

10. The device according to one of the preceding claims, which is utilized with a photosensitizer the activation of which produces substances capable of destroying cells.

Fig. 1

Fig. 2

Fig. 3

Fig. 4

Fig. 5

Fig. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2431072 A1
- EP 1637182 A1
- DE 19721489 A1
- WO 9906113 A
- EP 1808198 A1